# EUROPEAN PATENT SPECIFICATION

(11) **EP 3 987 051 B1**
(45) Date of publication and mention of the grant of the patent: **20.05.2026**
(21) Application number: 20736798.8
(22) Date of filing: 22.06.2020
(51) Int. Cl.: C12Q 1/66

(54) **RATIOMETRIC DETECTION OF LUCIFERASE ASSAYS USING A CALIBRATOR LUCIFERASE**
RATIOMETRISCHE DETEKTION VON LUCIFERASETESTS UNTER VERWENDUNG EINER KALIBRATORLUCIFERASE
DÉTECTION RATIOMÉTRIQUE D'ESSAIS DE LUCIFÉRASE À L'AIDE D'UNE LUCIFÉRASE D'ÉTALONNAGE

(30) Priority: 21.06.2019 US 201962864583 P; 29.08.2019 US 201962893322 P
(43) Date of publication of application: 27.04.2022
(73) Proprietor: Technische Universiteit Eindhoven, 5612 AE Eindhoven (NL)
(72) Inventor: MERKX, Maarten, 5612 AE Eindhoven (NL); NI, Yan, 5612 AE Eindhoven (NL); VAN IJZENDOORN, Leonardus Josephus, 5612 AE Eindhoven (NL)
(74) Representative: Algemeen Octrooi- en Merkenbureau B.V.
(86) International application number: PCT/NL2020/050406
(87) International publication number: WO 2020/256558

(56) References cited:
- WO-A1-2012/002507
- US-A1- 2015 191 769
- US-A1- 2016 376 332
- ALDO RODA ET AL: "Analytical strategies for improving the robustness and reproducibility of bioluminescent microbial bioreporters", ANALYTICAL AND BIOANALYTICAL CHEMISTRY, SPRINGER, BERLIN, DE, vol. 401, no. 1, 21 May 2011 (2011-05-21), pages 201 - 211, XP019920344, ISSN: 1618-2650, DOI: 10.1007/S00216-011-5091-3

## Description

### Technical field

The present invention relates to a bioluminescent assay for the ratiometric quantification of an analyte of interest comprising a detector luciferase that is responsive to the analyte of interest. The invention further relates to a method for quantifying an analyte of interest in a sample using the bioluminescent assay of the present invention and to the use of the bioluminescent assay of the present invention in a method for quantifying an analyte of interest in a sample.

### Background

Detection of biomarkers, such as protein biomarkers provides a useful tool for disease diagnosis and treatment monitoring. Classical immunoassays such as ELISA rely on a laborious procedure involving multiple washing and incubation steps. Alternative methodologies that empower single-step homogeneous immunoassays are more attractive and desirable for point-of-care tests that can be performed outside of the laboratory setting by non-expert users.

Split protein complementation is widely validated for studying protein-protein interactions and is an interesting alternative for developing sandwich immunoassays by placing the reporter fragments on a pair of antibodies that each bind the antigen targets. In such way, antigen binding brings the fragments in close proximity and thus allows reassembly of the active reporter, which quantitatively indicates the presence of the antigens. Split reporter enzymes, in particular split luciferases, are useful luminescent reporters that benefit from a great sensitivity. A particular useful split luciferase is split NanoLuc being a highly stable and bright, blue light producing luciferase that can be split into two fragments (so called Binary Technology also referred to as 'BiT'), an 18 kDa large BiT (LB) and a 1.3 kDa small BiT (SB), which were designed as a binary complementation reporter to study protein interactions.

Such split reporter enzymes, in particular split luciferases, have become important reporter systems to interrogate protein-protein interactions and high throughput drug screening. More in general, bioluminescent detection using luciferases is increasingly being used for in vivo imaging and point of care diagnostics. Unlike fluorescence detection, bioluminescence does not require external excitation, making it highly suitable for sensitive detection in complex media. A drawback of bioluminescent detection is that signal intensity is determined by many factors, including the concentration of both the luciferase and the substrate, product and environmental conditions including temperature, pH, and ionic strength. Since the substrate is turned over in time, the signal is time dependent, making quantitative measurements challenging.

Quantitative measurements based on bioluminescence activity require measurement of a calibration curve under conditions that closely mimic the conditions of the assay, including the amount of active enzyme, buffer conditions, substrate concentration, and temperature and incubation time. However, in many applications these parameters are not exactly known or cannot be easily controlled. In particular the time dependence of the luminescent signal is difficult to control, especially at higher luciferase activities. One remedy has been to choose assay conditions such that the luciferase activity is kept low, either by decreasing the enzyme concentration or by using caged substrates which result in a slow release of active substrate over time. However, these solutions are far from ideal, as the former limits the range of luciferase concentrations and signal intensity, while the latter depends on yet another time-dependent process. For this reason, bioluminescent assays have so far been semi-quantitative at best, making them popular for screening-based approaches but more challenging for accurate analytical applications.

Aldo Roda et al., Analytical strategies for improving the robustness and reproducibility of bioluminescent microbial bioreporters, Anal. Bioanal. Chem. 401(1), 201-211 (2011) discloses the use of different test and control luciferases that act on the same substrate but emit light at different wavelengths.

### Summary of the invention

The invention provides a method to allow internal calibration of bioluminescent assays based on (split) luciferases, which allows these assays to be used for quantitative measurements. The invention provides a solution by calibrating the bioluminescence intensity of a first luciferase (also referred to herein as a 'reporter luciferase' or a 'detector luciferase') to that of a second luciferase (also referred to herein as a 'calibrator luciferase') that is added to the same sample, uses the same luciferase domain and substrate, but emits light at a different wavelength.

The idea is to include in the assay a variant, i.e. the calibrator luciferase, of the luciferase used in the assay that is functionalized with a fluorescent acceptor. This calibrator luciferase contains the same luciferase domain as is used in the assay, but its energy is transferred to the fluorescent acceptor, resulting in emission at a different wavelength as the detector luciferase used in the assay. Since the calibrator luciferase does not respond to the target analyte, its activity (as determined by measuring the emission at the wavelength of the fluorescent acceptor) can be used to normalize the emission of the detector luciferase. This invention provides a generic solution to make bioluminescent assays and imaging more quantitative using ratiometric detection of the detector luciferase emission at one wavelength relative to the calibrator luciferase emission detected at a different wavelength. The assumption behind this approach is that variations in substrate concentration, product inhibition, matrix composition and temperature will affect the detector luciferase and the calibrator luciferase to the same extend.

### Description

The present invention provides a bioluminescent assay for the ratiometric quantification of an analyte of interest. In order to provide a time and concentration independent quantification of an analyte of interest, the bioluminescent assay of the present invention comprises a detector luciferase that is reactive to a substrate to emit light at a first wavelength and wherein the detector luciferase is responsive to the analyte of interest. The bioluminescent assay of the present invention further comprises a calibrator luciferase that is functionalized with a fluorescent acceptor to allow energy transfer from the luciferase domain of the calibrator luciferase to the fluorescent acceptor. The calibrator luciferase of the present invention is non-responsive to the analyte of interest, and is reactive to the same substrate to emit light at a second wavelength which second wavelength is different from the first wavelength emitted by the detector luciferase. The calibrator luciferase used in the bioluminescent assay of the present invention has the same luciferase domain as the detector luciferase.

By providing the bioluminescent assay of the present invention, the calibrator luciferase is able to catalyse the oxidation of the same substrate as is catalysed by the detector luciferase. For example, in case the detector luciferase is a NanoLuc luciferase that catalyses the oxidation of the substrate furimazine (to generate blue light), the corresponding luciferase domain of the calibrator luciferase, e.g. a NanoLuc-mNeonGreen fusion protein, catalyses oxidation of the same substrate furimazine (to generate green light). By using a detector luciferase and a calibrator luciferase having the same luciferase domain, deviations in, for example, substrate concentrations are automatically corrected for by calibrating the ratio of measured bioluminescence intensity of the detector luciferase and measured bioluminescence intensity of the calibrator luciferase. By using a detector luciferase according to the present invention the emission ratio of measured bioluminescence intensity of the detector luciferase and measured bioluminescence intensity of the calibrator luciferase remains stable over time and can therefore be reliably detected as long as sufficient light is emitted by both the detector luciferase and calibrator luciferase.

In order to emit light at a different wavelength than the detector luciferase the calibrator luciferase is functionalized with a fluorescent acceptor to allow energy transfer from the luciferase domain of the calibrator luciferase to the fluorescent acceptor. Such a fluorescent acceptor may be selected from the group consisting of an mNeonGreen protein, a fluorescent protein, a Cy3, a fluorescent dye, a fluorescent quantum dot, a fluorescent nanoparticle, or a carbon dot. For example, in case a NanoLuc luciferase detector and calibrator luciferase is selected, in order to provide a blue/green shifted calibrator luciferase, the preferred fluorescent acceptor is an mNeonGreen protein. Alternatively, in order to provide a blue/red shifted NanoLuc calibrator luciferase, the preferred fluorescent acceptor is a Cy3.

In order to avoid interference with the detector activity of the detector luciferase, the calibrator luciferase is non-responsive to the analyte of interest. Preferably, in order to provide an optimal system the calibrator luciferase is responsive to the same components comprised in a sample to be analysed as the detector luciferase is responsive to, except for the responsiveness to the analyte of interest. By providing such calibrator luciferase, any response to other analytes or components comprised in the sample by the detector luciferase is automatically corrected for by calibrating the ratio of measured bioluminescence intensity of the detector luciferase and measured bioluminescence intensity of the calibrator luciferase.

Although the use of a NanoLuc luciferase domain is preferred due to its bright blue emission characteristics, other luciferase domains may be suitable as well. For example, the luciferase domain of the detector luciferase and calibrator luciferase may be selected from the group consisting of luciferase domains of NanoLuc luciferase, Firefly luciferase, Renilla luciferase, Gaussia luciferase, TurboLuc luciferase and Aluc luciferase.

The analyte of interest of the sample to be analysed, may be any analyte of interest, and may be relevant for diagnosing or treatment monitoring. However, other applications, such as the detection of food toxins, water quality, and the like, may also be possible. Preferred analytes of interest may be selected from the group consisting of an antigen, an antibody, and a ligand.

Any type of bioluminescent assay may be used in order to provide the time-independent and concentration-independent quantification assay of the present invention. As long as a calibrator luciferase is selected having the same luciferase domain as the detector luciferase, detection and quantification of the analyte of interest of a sample to be analysed can be performed in a reliable manner. For example, the bioluminescent assay may be a bioluminescent sandwich immunoassay, or other types of immunoassays such as competitive immunoassays, sensor proteins based on allosteric modulation of luciferase activity or reversible control a luciferase inhibition, transcription regulation assays, assays screening for protein-protein interactions, DNA or RNA detection assays.

In an aspect of the present invention, the invention relates to the use of the bioluminescent assay according to the present invention in a method for quantifying an analyte of interest in a sample.

In a further aspect of the present invention, the invention relates to the use of a calibrator luciferase in the bioluminescent assay according to the present invention in a method for quantifying an analyte of interest in a sample, wherein the calibrator luciferase is functionalized with a fluorescent acceptor to allow energy transfer from the luciferase domain of the calibrator luciferase to the fluorescent acceptor.

In another aspect of the present invention, the invention relates to a method for quantifying an analyte of interest in a sample. The method comprises the steps of:
i) providing a sample comprising the analyte of interest;
ii) providing a detector luciferase and a calibrator luciferase, wherein the detector luciferase:
   - is responsive to the analyte of interest; and
   - is reactive to a substrate to emit light at a first wavelength,
iii) measuring the bioluminescence intensity of the detector luciferase and the bioluminescence intensity of the calibrator luciferase for the sample provided in step i);
iv) calibrating the ratio of the measured bioluminescence intensity of the detector luciferase and the bioluminescence intensity of the calibrator luciferase; and
v) quantifying the analyte of interest based on the ratio calibrated in step iv),
wherein the calibrator luciferase is functionalized with a fluorescent acceptor to allow energy transfer from the luciferase domain of the calibrator luciferase to the fluorescent acceptor, said calibrator luciferase:
- is non-responsive to the analyte of interest; and
- is reactive to the same substrate to emit light at a second wavelength which second wavelength is different from the first wavelength emitted by the detector luciferase, and wherein the detector luciferase and the calibrator luciferase have the same luciferase domain.

### Embodiments

In one embodiment of the present invention the present invention relates to a bioluminescent sandwich immune assay that can be performed directly in solution without any washing or incubation steps. In such assay, two antibodies that recognize different epitopes on the analyte of interest are conjugated to the large or small portions of split Nanoluc luciferase by photo-cross-linking of a protein G domain fused to the split NanoLuc part via a flexible peptide linker. In the presence of the analyte of interest a ternary complex is formed of the analyte with the two antibodies, allowing complementation of the two split luciferase parts and reconstitution of luciferase activity. This system by itself generates an intensiometric signal where the intensity of the blue light emitted by the reconstituted luciferase is a measure of the amount of analyte. However, the intensity of the blue luminescence is not constant and decreases in time, in particular for the high analyte concentrations. The problem was resolved by spiking in a low concentration of a NanoLuc-mNeonGreen fusion protein, which does not emit in the blue (460nm) but emits green light (515 nm). The ratio of green and blue light was found to be stable in time, allowing the emission ratio to be used as a reliable measure of the analyte concentration. Ratiometric detection in combination with split NanoLuc complementation provides a very attractive and fast alternative to the currently used heterogeneous sandwich immunoassay. The method may be used for the detection of cardiac troponin I (a marker for heart attack), C-reactive protein and anti-cetuximab antibodies, however, other applications of the method may be suitable as well including the detection of other antibodies and protein biomarkers.

Other interesting applications of split reporter luciferases is in high throughput detection of protein-protein interactions. In general, the method provides a convenient and much more robust method to calibrate any bioluminescent assay.

Embodiments of the invention can be applied as a sensor strategy to develop assays and point-of-care diagnostics for several other interesting biomarkers and combine the assay with paper- or thread based diagnostic devices, for which ratiometric detection is of key importance. The method itself can be further varied by exploring calibrator variants with a more red-shifted emission signal (better separation from signal of detector luciferase). The invention can also be applied more broadly beyond in vitro assays (with luciferases), as it provides a general principle to calibrate an intensiometric bioluminescent signal. Applications envisioned include the use in cell-based high throughput screening, transcriptional reporter assays, (in vivo) bioluminescent imaging applications and novel ways to develop ratiometric bioluminescent sensor proteins.

Although the examples provided herein involve a split luciferase, the detector luciferase could be any luciferase whose activity or concentration is regulated by of the presence of an analyte. With that, the invention is also applicable to some non-split luciferases.

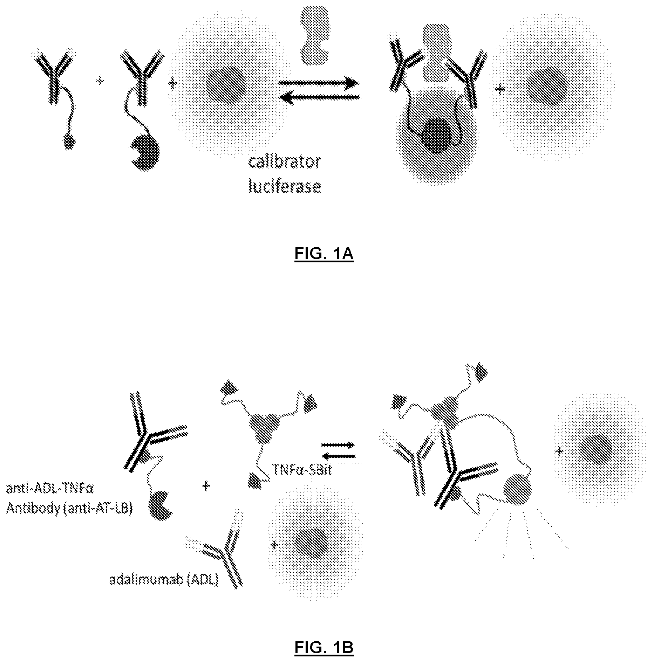

The present invention relates to an alternative ratiometric bioluminescent sandwich immunoassay format based on complementation of a split reporter luciferase, where the two fragments of the luciferase are conjugated to a pair of antibodies that each recognize a different epitope on the target molecule, allowing for a homogeneous sandwich immunoassay in solution (see also: Ni, Y. and M. Merkx, Ratiometric detection of luciferase assays using a calibrator luciferase. provisional patent application 62/864583, manuscript in preparation, 2020). A protein G-mediated photo-conjugation strategy (see also: Hui, J.Z., et al., LASIC: Light Activated Site-Specific Conjugation of Native IgGs. Bioconjugate Chem. 2015, 26, 8, p. 1456-1460) was introduced to allow site-specific covalent bond formation between native antibodies and split NanoLuc fragments (see also: Dixon, A.S., et al., NanoLuc Complementation Reporter Optimized for Accurate Measurement of Protein Interactions in Cells. ACS Chemical Biology, 2016. 11(2): p. 400-408), providing a generic and straightforward method applicable to a wide range of commercially available monoclonal antibodies (Figure 1A). Moreover, the system was made ratiometric by introducing NanoLuc-mNeonGreen (see also: Suzuki, K., et al., Five colour variants of bright luminescent protein for real-time multicolour bioimaging. Nature Communications, 2016. 7: p. 1-10) as a calibrator luciferase to allow for time and concentration independent measurements. While this system could also be used for antibody detection, in that case the assay showed a relatively small increase in luciferase complementation and a bell-shaped antibody response curve (also known as the hook effect). These properties are inherent to a solution sandwich immunoassay targeting two antigen binding sites within the same detection antibody, and restrict the analytical performance of the assay (see also: Ni, Y. and M. Merkx, Ratiometric detection of luciferase assays using a calibrator luciferase. provisional patent application 62/864583, manuscript in preparation, 2020). The alternative assay format successfully addresses these shortcomings. Key to this new assay format is that one part of the split luciferase is genetically fused to the target antigen, whereas the complementary part is fused to a monoclonal antibody that specifically recognizes the antibody or the antibody-antigen complex. Proof of principle for this assay format is provided by developing a ratiometric bioluminescent assay for the detection of adalimumab.

In a further embodiment the invention relates to ratiometric bioluminescent immunoassays for TNFα binding antibodies. Antibodies binding TNFα, a pro-inflammatory cytokine that is overexpressed in inflammatory diseases, are an important example of therapeutic antibodies. TNFα actually forms a homotrimer in solution and therapeutic antibodies such as adalimumab bind TNFα by recognizing a complex discontinuous epitope formed by the interface of two monomers. Therapeutic antibodies that target TNFα such as infliximab and adalimumab have been highly successful in treating diseases such as such as Rheumatic Arthritis and Inflammatory Bowel Disease, and represent some of the top-selling drugs.

### Examples

### Example 1. Bioluminescent sandwich immune assay for the detection of cardiac troponin I, C-reactive protein and anti-cetuximab antibodies

### Cloning

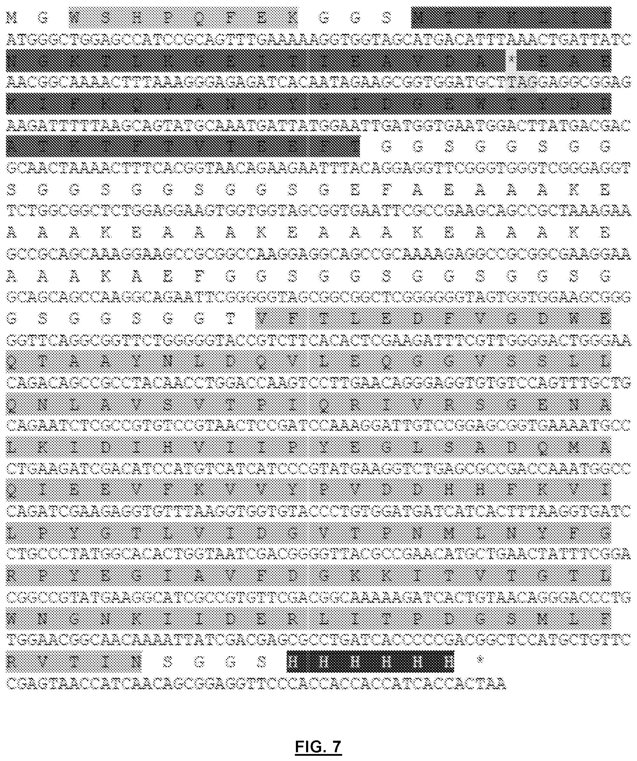

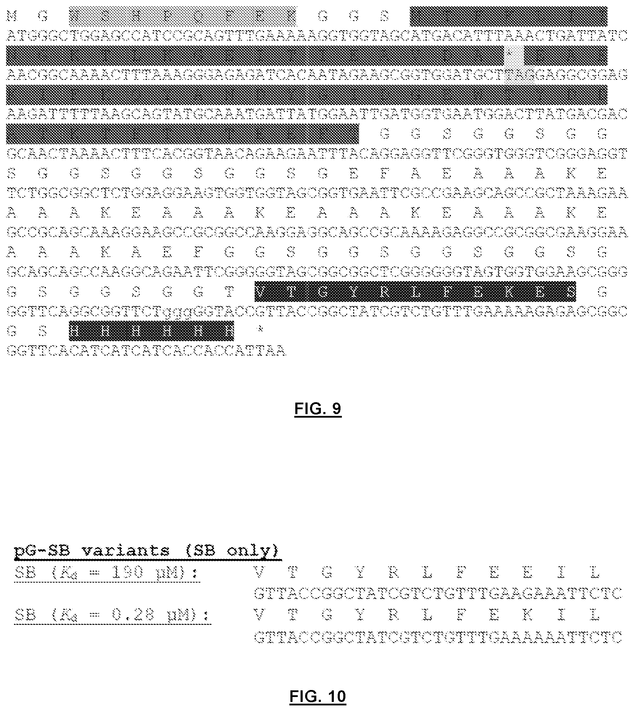

The pET28a(+) vectors containing DNA encoding protein G-SB (pG-SB) and protein G-LB (pG-LB) were ordered from GenScript. Site directed mutagenesis to change SB sequences were carried using the QuikChange Lightning Site-Directed Mutagenesis kit (Agilent technologies) using specific primers. All cloning and mutagenesis results were confirmed by Sanger sequencing (StarSEQ). Figures 7, 9 and 10 show the DNA and corresponding amino acid sequences of pG-SB (variants) and pG-LB.

### Protein expression

The pET28a plasmids encoding pG-LB or pG-SB were co-transformed into *E. coli* BL21 (DE3) together with a pEVOL vector encoding a tRNA/tRNA synthetase pair in order to incorporate para-benzoylphenylalanine (pBPA). The pEVOL vector was a gift from Peter Schultz (Addgene plasmid # 31186). Cells were cultured in 2YT medium (16 g peptone, 5 g NaCl, 10 g yeast extract per liter) containing 30 µg/mL kanamycin and 25 µg/mL chloramphenicol. Protein expression was induced using 0.1 mM IPTG and 0.2% arabinose in the presence of 1 mM pBPA (Bachem, F-2800.0001). After overnight expression, cells were harvested and lysed using the Bugbuster reagent (Novagen). Proteins were purified using Ni-NTA affinity chromatography followed by Strep-Tactin purification according to the manufacturer's instructions. Protein purity was confirmed by SDS-PAGE. Correct incorporation of pBPA was confirmed by Q-ToF LC-MS. Purified proteins were stored at -80°C until use.

The NanoLuc-mNeonGreen fusion protein was prepared as described previously (Suzuki, K., et al., Five colour variants of bright luminescent protein for real-time multicolour bioimaging. Nature Communications, 2016. 7: p. 1-10). Proteins were purified using Ni-NTA affinity chromatography and Strep-Tactin purification. Purified proteins were stored at -80°C until use.

### Photo-conjugation

Cardiac Troponin I antibodies 19C7 (4T21) and 4C2 (4T21), and C-reactive protein antibodies C6 (4C28cc) and C135 (4C28) were ordered from Hytest. Therapeutic antibody cetuximab was obtained via the Catherina hospital pharmacy in Eindhoven, the Netherlands. Photo-conjugation reactions were performed using a Promed UVL-30 UV light source (4×9 watt). Samples containing antibody and pG-LB or pG-SB in PBS buffer (pH7.4) were illuminated with 365 nm light for 30 to 180 minutes. The samples were kept on ice during photo-conjugation. The photo-conjugated products were further purified using Ni-NTA spin columns followed by PD G10 desalting columns or protein G spin columns.

### Luminescent assays

Cardiac Troponin I (8T53) and C-reactive protein (8C72) were ordered from Hytest. Anti-cetuximab (HCA221) was ordered from Bio-rad. Intensiometric assays were performed at sensor proteins concentrations of 0.1-10 nM in a total volume of 15 µL in PerkinElmer flat white 384-well Optiplate. After incubation of sensor proteins and analytes for 0.5 hours, NanoGlo substrate (Promega, N1110) was added at a final dilution of 300- to 1000-fold. Luminescence intensity was recorded on Tecan Spark 10M plate reader with an integration time of 100 ms. For ratiometric assays, 1-10 pM NanoLuc-mNeonGreen fusion protein was added into the samples and luminescence spectra were recorded between 398 nm and 653 nm with a step size of 15 nm, a bandwidth of 25 nm and an integration time of 1000 ms.

The luminescence signal was also recorded by using a digital camera. The plate was placed into a styrofoam box to exclude the surrounding light. The pictures were taken through a hole in the box using a SONY DSC-RX100 digital camera with exposure times of 30 s, F value of 1.8 and ISO value of 1600-3200. The images were analyzed by using ImageJ to calculate the absolute intensity and ratio values between the average intensity in the blue and green color channel.

### Example 1a. Sensor design and characterization (cardiac troponin I)

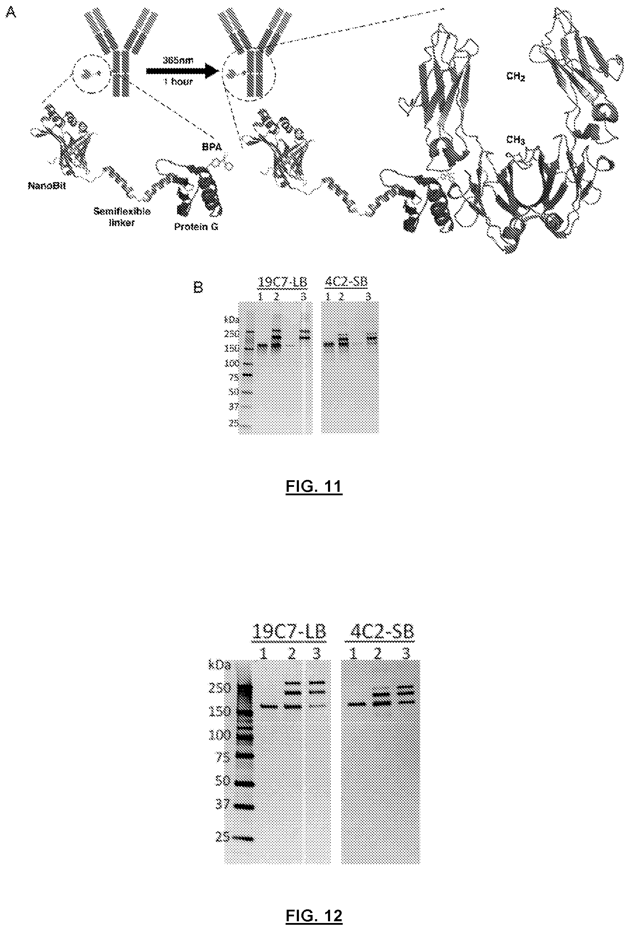

In order to establish a proof-of-concept, cardiac troponin I (cTnl) as a target antigen was chosen which is an important marker for cardiac damage and requires highly sensitive detection at pM concentrations. An LB fragment of split NanoLuc and an SB fragment with a *K_{d}* of 2.5 µM, respectively, were fused to protein G via a semi-flexible linker. A His-tag at N-terminus and a strep-tag at C-terminus were included to facilitate the purification of the fusion proteins. The plasmid contained a TAG amber stop codon at position 24 in protein G, and co-expression with the orthogonal tRNA synthetase/tRNA pair allowed the incorporation of the unnatural amino acid para-benzyol-phenylalanine (BPA), a photo-reactive group, at the desired position (see also: Hui, J.Z., et al., LASIC: Light Activated Site-Specific Conjugation of Native IgGs. Bioconjugate Chem. 2015, 26, 8, p. 1456-1460). The BPA incorporated protein G domain can be crosslinked to the Fc domain of an antibody with 365 nm light illumination. The purified pG-LB and pG-SB proteins were photo-conjugated to a pair of anti-cTnl antibodies, 19C7 and 4C2, that are compatible in sandwich immunoassays (figure 11A). One feature of protein G-based conjugation is that both IgG heavy chains can be modified, so a mixture of mono-conjugated, di-conjugated and non-conjugated antibodies as well as pG-LB/SB was obtained. The conjugation efficiency was affected by using various molar ratios of adapter protein to antibody (figure 12). Since the presence of pG-LB and pG-SB contributes to the background signal, we used an equal molar ratio of protein to antibody to minimize the unconjugated pG-LB and pG-SB in the mixture. A step of Ni affinity chromatography was used to remove the unconjugated antibody (figure 11B).

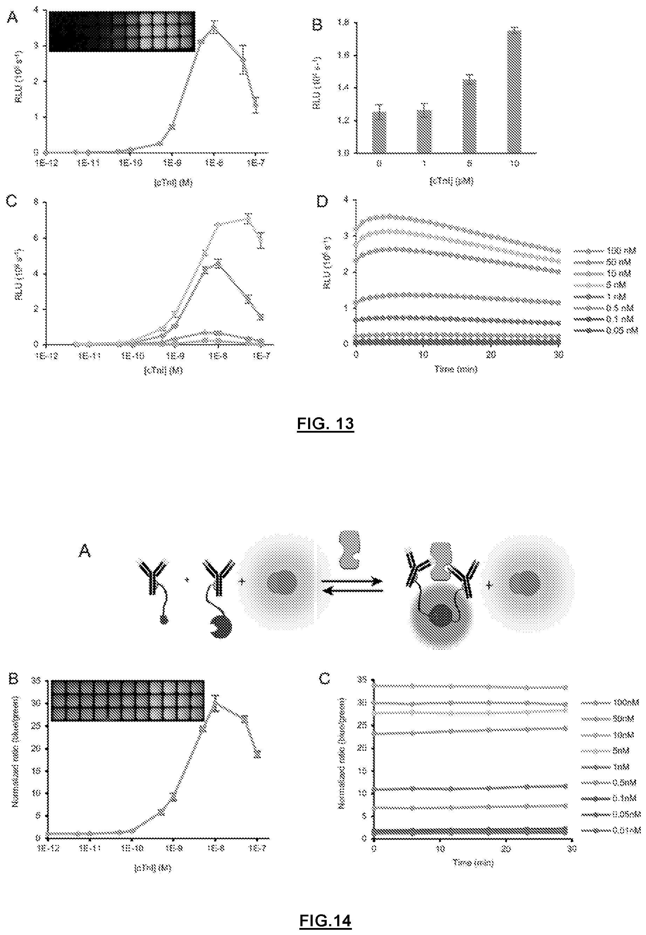

When 1 nM of the purified antibody-conjugated sensor proteins were incubated with cTnl in the pico- to nanomolar regime, the recorded luminescence intensity followed a bell-shaped, cTnl-dependent curve with hook effect at very high concentrations of cTnl (figure 13A). The maximum luminescence intensity observed at 10 nM of cTnl was more than 300-fold higher compared to the blank. The limit of detection was determined to be 5 pM (figure 13B). The signal response could be modulated by varying the concentrations of the sensor proteins (figure 13C). The use of higher concentrations of 4C2-SB could effectively shift the "hook" to relatively higher analyte concentrations. The maximum intensity was obtained at 50 nM of cTnl by using 1 nM of 19C7-LB and 10 nM of 4C2-SB. A higher S/B ratio at low analyte concentrations was obtained by using 0.1 nM 19C7-LB and 1 nM 4C2-SB, although these intensities were too low to be detected by a digital camera. Therefore, 1 nM of each sensor protein was employed in the further experiments. The effect of the split NanoLuc interaction affinities on the signal response was further investigated. The other two SB variants with *K_{d}* of 190 µM and 0.28 µM were tested for this purpose. The highest signal intensity was obtained using SB with *K_{d}* of 2.5 µM with a highest S/B ratio (figure 14).

One drawback of using the absolute luminescence intensity is that the signal intensity depends on many factors including substrate concentration, pH, temperature and ionic strength and typically decreases in time as a result of substrate turn-over. Indeed, the monitoring of the absolute luminescence intensity for a period of 30 minutes revealed that the signal changed in time (figure 13D). This is particularly problematic for point-of-care applications where it may be difficult to carefully calibrate the assay for variations in sensor concentration, substrate turn-over and environmental conditions. A ratiometric system was further developed by adding an additional calibrator luciferase. The assay mixtures were spiked with a bioluminescent NanoLuc-mNeonGreen fusion protein which can catalyse oxidation of the same substrate furimazine and generate green light (Suzuki, K., et al., Five colour variants of bright luminescent protein for real-time multicolour bioimaging. Nature Communications, 2016. 7: p. 1-10). In such way, the emission ratio as a function of antigen concentration negates the influence of the substrate concentration and the reaction conditions. As shown in figure 14A, the ratiometric system displayed a similar antigen dose-dependency as that obtained by the intensiometric assays. More interestingly, the emission ratio remains stable over time (figure 14B) and therefore can be reliably detected as long as sufficient light is emitted. The time-independency of the ratiometric system represents an important aspect of sensor performance for practical applications.

### Example 1b. Sensor design and characterization (C-reactive protein)

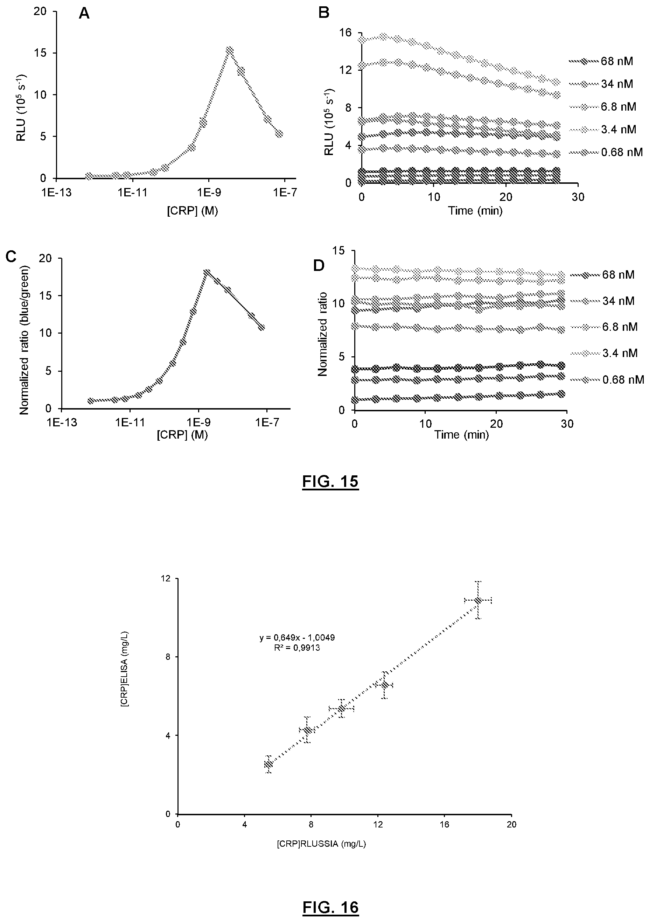

The assay format of example 2 was also applied for the detection of the inflammatory marker C-reactive protein (CRP). The pG-SB and pG-LB proteins were conjugated to a pair of anti-CRP antibodies C135 (mlgG2b) and C6 (mlgG2a), respectively. Following the successful photo-conjugation of antibodies with pG-SB and pG-LB and one-step nickel affinity purification, the intensiometric assays for CRP were carried out, yielding a maximal S/B ratio of 56 (figure 15A). Addition of the NL-mNG calibrator made the assays less fluctuant over time without influence on the detection limit and regime (figure 15C and figure 15D). The maximal ratio change of 18 and a LOD of 3 pM were achieved in the ratiometric assays by adding 2 pM of NL-mNG calibrator.

### Assay evaluation

The analytical performance of the assay was compared with a commercially available ELISA for quantification of CRP in human blood plasma. The test CRP samples were prepared at low mg L⁻¹ level referring to high-sensitivity CRP (hsCRP) which is clinically used for cardiovascular risk assessment. Due to the high sensitivity of our assay, the test blood plasma samples were diluted 50 times in buffer and then measured by comparing the measured blue/green ratio with the calibration curve obtained in figure 15C. In parallel, the CRP concentration in the test samples were determined by ELISA after appropriate dilution. A good correlation (R² = 0.9906) was observed between the ELISA and our assay (figure 16), pointing to the accuracy of our assay and its good potential for the analysis of clinical samples. It is worth mentioning that the simple "mix-and-measure" workflow of our assay eliminates the need for multiple wash steps and thus substantially reduces total assay times to less than 1 hour compared to ELISA.

### Example 1c. Sensor design and characterization (anti-cetuximab)

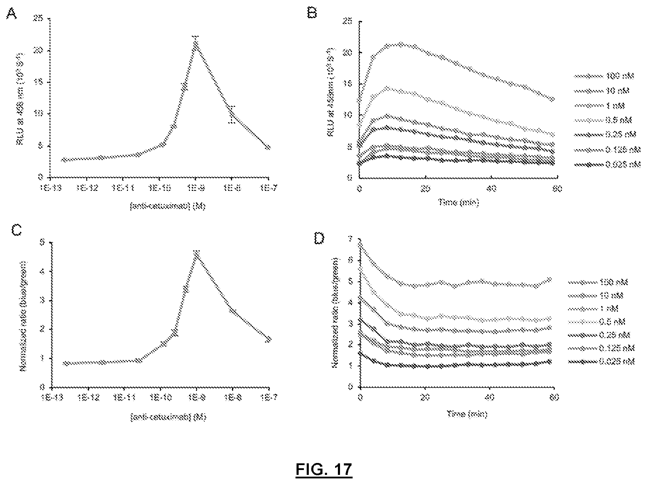

The generic sensor format of example 1 was investigated whether it could be extended to anti-antibodies monitoring. The administration of therapeutic antibodies for cancer or inflammation therapy can induce an immune response and lead to the production of anti-antibodies inactivating the therapeutic effects of the treatment. Therefore development of a simple and fast assay to detect anti-antibodies is important for assessing immunogenicity to therapeutic antibodies. An anti-cancer therapeutic antibody cetuximab was chosen as the detecting molecule which was photo-conjugated separately with pG-LB and pG-SB. The split NanoLuc functionalized cetuximab was then utilized for the luminescent detection of anti-cetuximab and a bell-shaped dose-dependency curve was obtained with the maximum S/B ratio of 8 (figure 17A). Addition of NanoLuc-mNeonGreen fusion protein in the ratiometric system yielded a similar assay curve (figure 17C) but more stable sensor signal of emission ratio over time (figure 17D).

### Conclusion

A generic homogenous immunoassay format based on the complementation of split Nanoluc luciferase was developed for direct detection of protein biomarkers in solution. Proof of concept was provided by successful detection of cTnl at pM concentrations. The high modularity of the sensor format enables assay of various protein targets by placing the split NanoLuc fragments on the target-binding antibodies. Moreover, a ratiometric assay system was developed by adding a calibrator luciferase to allow for time and substrate concentration independent measurements.

### Example 2. Ratiometric bioluminescent immunoassays for TNFα binding antibodies

### Cloning

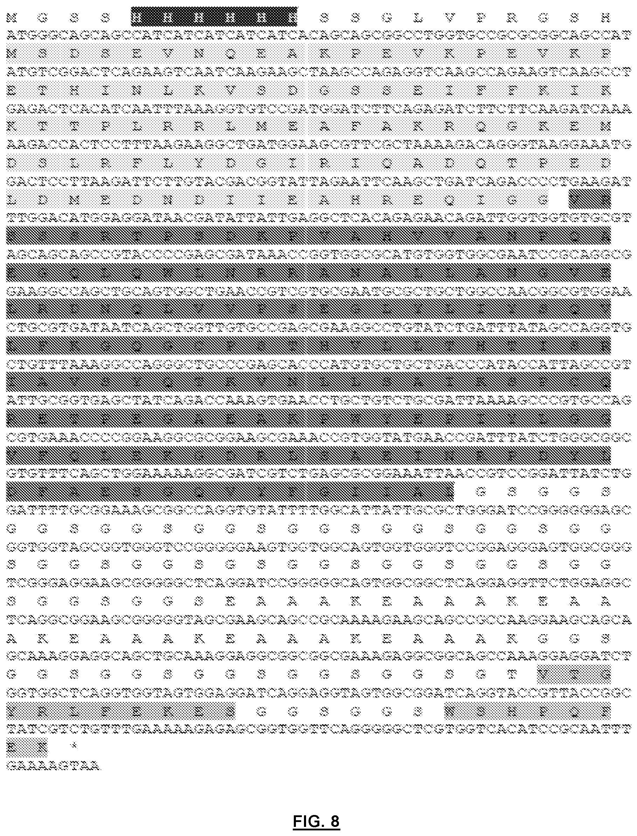

The pET28a(+) vector containing DNA encoding protein G-LargeBit (pG-LB) and the pET28a(+) vector containing DNA encoding His-SUMO-TNFα-linker-SB were ordered from GenScript. Figure 7 and Figure 8 show the DNA and corresponding amino acid sequences of pG-LB and SUMO-TNFα-SB, respectively.

### Protein expression and purification

The pET28a plasmid encoding pG-LB was co-transformed into *E. coli* BL21 (DE3) together with a pEVOL vector encoding a tRNA/tRNA synthetase pair in order to incorporate para-benzoyl-phenylalanine (pBPA). The pEVOL vector was a gift from Peter Schultz (Addgene plasmid # 31186). Cells were cultured in 2YT medium (16 g peptone, 5 g NaCl, 10 g yeast extract per liter) containing 30 µg/mL kanamycin and 25 µg/mL chloramphenicol. Protein expression was induced using 0.1 mM IPTG and 0.2% arabinose in the presence of 1 mM pBPA (Bachem, F-2800.0001). After overnight expression, cells were harvested and lysed using the Bugbuster reagent (Novagen). Proteins were purified using Ni-NTA affinity chromatography followed by Strep-Tactin purification according to the manufacturer's instructions. Protein purity was confirmed by SDS-PAGE. Correct incorporation of pBPA was confirmed by Q-ToF LC-MS. Purified proteins were stored at -80°C until use.

The pET28a plasmid encoding SUMO-TNFα-SB fusion protein was transformed into *E. coli* BL21 (DE3). Cells were cultured in LB medium (10 g peptone, 10 g NaCL, 5 g yeast extract per liter) containing 30 µg/mL kanamycin. Protein expression was induced using 0.1 mM IPTG. Cells were harvested after overnight expression and were resuspended in Binding Buffer (40 mM Tris-HCl, 125 mM NaCl, 5 mM imidazole, pH 8.0) containing Benzonase enzyme. Cell disruption was then performed by ultrasonication on ice with 5 pulses of 20 s set to an amplitude of 50%. Proteins were first purified using Ni-NTA affinity chromatography at 4°C. The His-SUMO-tag was cleaved from TNFα-SB by adding 2 µL of SUMO protease to the 1.5 mL elution fractions and dialyzing the solution overnight at 4°C against SUMO protease reaction buffer (20 mM Tris-HCl, 150 mM NaCl, 1 mM DTT, pH 8.0) using a 5 kDa dialysis membrane. Cleaved TNFα-SB was obtained using Ni-NTA affinity chromatography to remove the cleaved His-SUMO-tag. Protein purity was confirmed by SDS-PAGE. Purified proteins were stored at -80°C until use.

The NanoLuc-mNeonGreen fusion protein was prepared as described previously (Suzuki, K., et al., Five colour variants of bright luminescent protein for real-time multicolour bioimaging. Nature Communications, 2016. 7: p. 1-10). Proteins were purified using Ni-NTA affinity chromatography and Strep-Tactin purification. Purified proteins were stored at -80°C until use.

### Photo-conjugation

Anti-adalimumab/TNFα monoclonal antibody (ATantibody) was obtained from BioRad (HCA207). Photo-conjugation reactions were performed using a Promed UVL-30 UV light source (4×9 watt). Samples containing antibody and 2 equivalents of pG-LB in PBS buffer (pH 7.4) were illuminated with 365 nm light for 2 hours. The samples were kept on ice during photo-conjugation. The photo-conjugated products contained a mixture of unconjugated pG-LB and AT antibody conjugated with one or two copies of pG-LB protein and were used without further purification.

### Luminescent assays

Adalimumab was ordered from Gentaur (A1048-100). Intensiometric assays were performed at HCA207-pG-LB concentrations of 2.5-30 nM and TNFα-SB concentrations of 25-300 nM in a total volume of 15 µL in Greiner flat white 384-well plates. After incubation of sensor proteins and analytes for 0.5 hours, NanoGlo substrate (Promega, N1110) was added at a final dilution of 500-fold. Luminescence intensity was recorded on Tecan Spark 10M plate reader with an integration time of 100 ms. For ratiometric assays, 10-100 pM NanoLuc-mNeonGreen fusion protein was added into the samples and luminescence spectra were recorded between 398 nm and 653 nm with a step size of 15 nm, a bandwidth of 25 nm and an integration time of 100 ms. Experiments were done in buffer (PBS with 1 mg/ml BSA, pH 7.4) or in blood plasma diluted with PBS/BSA buffer.

### Sensor design synthesis of sensor components

In order to establish a proof-of-concept, adalimumab (ADL) was chosen as a target antibody. Adalimumab is currently the most widely used anti-TNFα antibody, and a representative of a family of TNFα blocking biopharmaceuticals widely used in treating inflammatory diseases that also include infliximab and its biosimilars, and hybrid proteins containing non-antibody receptor domains. The sensor according to this example has two parts; (1) a TNFα fusion protein in which the C-terminus of TNFα is linked via a flexible linker to a SB, and (2) a monoclonal antibody that binds the ADL-TNFα complex that is functionalized with a LB fragment using a photo-cross-linkable protein-linker-LB fusion protein previously reported. The high affinity of ADL for the native TNFα trimer (*K_{d}* = 0.11 nM) ensures that all ADL will be form a complex with TNFα when an excess of the latter is used in the assay. The anti ADL-TNFα antibody used here was reported to bind the complex with a *K_{d}* of 67 nM (https://www.bio-rad-antibodies.com/anti-adalimumab-antibody-humira.html), which allowed the assay to be sensitive in the concentration range that is of interest for therapeutic drug monitoring (0.1-22 mg/L or 1-200 nM).

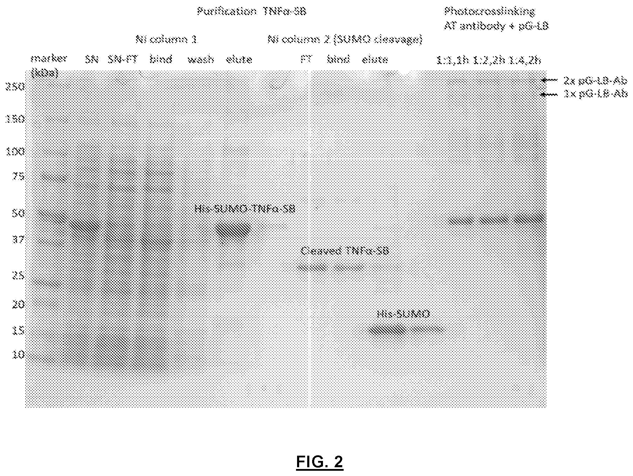

The DNA sequence of TNFα was taken from Hoffmann et al. (Recombinant production of bioactive human TNF-α by SUMO-fusion system - High yields from shake-flask culture. Protein Expression and Purification, 5 2010. 72(2): p. 238-243), who used an N-terminal His-SUMO-tag to obtain a high yield of the properly folded protein. This DNA sequence was cloned into a pET28a vector such that the C-terminus of TNFα is fused via a long, flexible glycine-serine linker to a SB variant with moderate affinity for the LB fragment (*K_{d}* of 2.5 µM). Attachment to the C-terminus of the protein should allow formation of the native trimeric complex without any steric hindrance of the fragment. Moreover, it was also important to provide sufficient steric freedom between both fragments such that the luciferase complementation could take place. The His-SUMO-TNFα-SB fusion protein was recombinantly expressed in *E. coli* and purified in good yield using nickel-affinity chromatography (see: figure 2). The His-SUMO tag was removed by overnight incubation of His-SUMO-TNFα-SB with SUMO protease, followed by a second nickel affinity chromatography step to separate TNFα-SB from His-SUMO and uncleaved HIS-SUMO-TNFα-SB protein. The flow through of this column contained essentially pure TNFα-SB fusion of the expected molecular weights (28 kDa). Conjugation of LB to the anti ADL-TNFα antibody was achieved by photo-crosslinking the antibody with 2 equivalents of pG-LB protein for 2 hours using 365 nm on ice. SDS-PAGE analysis revealed successful conjugation of most of the antibody with 1 or 2 copies of the pG-LB protein. The conjugation product was not further purified and used directly in the bioluminescent assay.

### Proof-of-principle and effect of sensor concentration

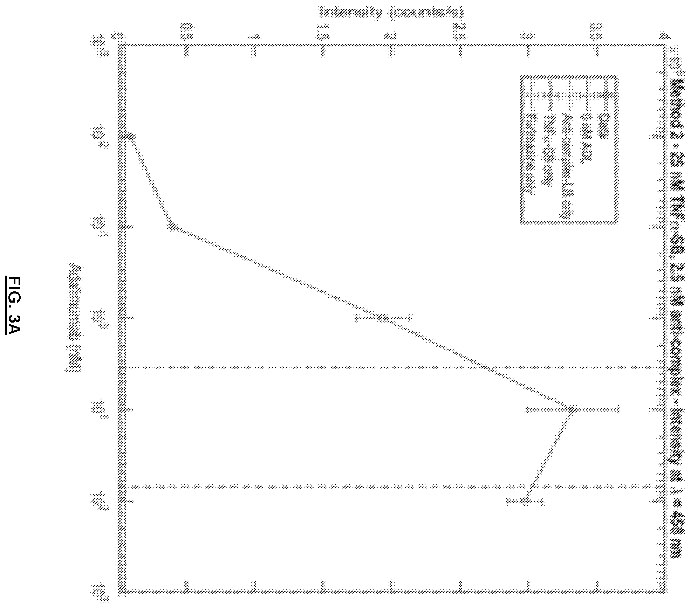

To test the feasibility of the new sensor principle we performed an adalimumab titration experiment in buffer using 2.5 nM anti-AT-LB and 25 nM TNFα-SB. A large, 80-fold increase in luminescence intensity was observed between 0.01 and 10 nM ADL, after which the intensity remained constant. This impressive increase in luminescence demonstrates that the principle of the assay works and that the TNFα-SB proteins folded correctly and self-assembled into their native trimeric structure. Next the concentration of anti-AT-LB and TNFα-SB was systematically changed to find optimal assay conditions with respect to the increase in signal increase and the ADL sensitivity. Figures 3A-B show that increasing the anti-AT-LB concentration increases both the signal and the background luminescence signal, without significantly affecting the dynamic range. Increasing the concentration of TNFα-SB from 100 to 300 nM does not affect the assay performance.

### Measurements in diluted blood plasma and ratiometric detection.

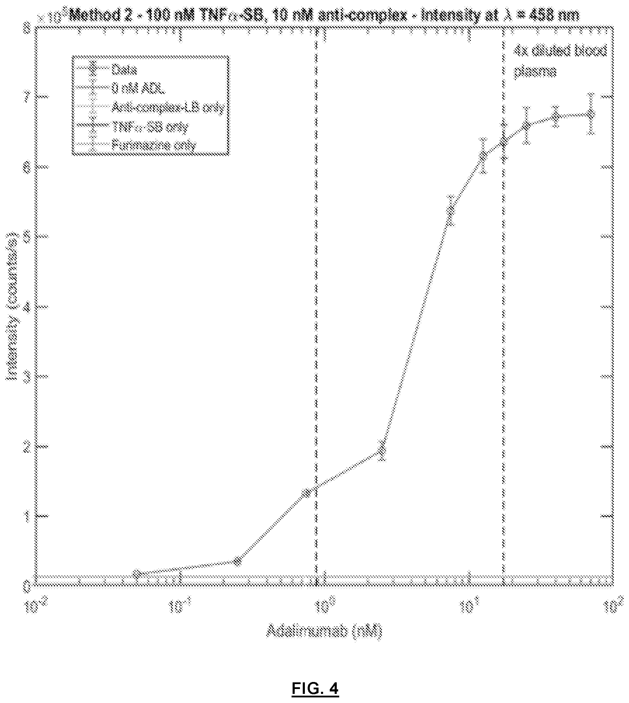

Ideally the assay should be able to measure ADL concentrations between 3 and 70 nM in blood plasma. Since the assay in buffer is most sensitive between 0.1 and 10 nM ADL, a protocol was established in which the plasma sample was diluted with buffer in a 1:3 ratio, corresponding to a 4-fold dilution. Figure 4 shows the results of an ADL titration experiment in diluted blood plasma, where the concentrations of ADL on the X-axis represent the final concentrations after dilution. The response curve and dynamic range are very similar to that obtained in pure buffer, showing that the assay is not affected by the presence of 25% (v/v) of blood plasma.

The data shown thus far represent intensiometric data in which the absolute intensity of the blue luminescent signal is measured as a signal for complex formation. However, the absolute signal intensity not only depends on the amount of ADL, but is also affected by environmental parameters such as pH, temperature and ionic strength. Most importantly, however, the signal depends on the concentration of furimazine substrate, which means that the signal will decrease in time as a result of substrate turn-over, most significantly at higher ADL concentration. This is particularly problematic for point-of-care applications where it may be difficult to carefully calibrate the assay for variations in sensor concentration, substrate turn-over and environmental conditions. Recently a new approach to convert intensiometric luciferase-based assays was reported such as these into ratiometric assays by simply adding an additional calibrator luciferase. The assay mixtures were spiked with a bioluminescent NanoLuc-mNeonGreen fusion protein which can use the same substrate furimazine and generate green light (Ni, Y. and M. Merkx, Ratiometric detection of luciferase assays using a calibrator luciferase. provisional patent application 62/864583, manuscript in preparation, 2020). In this way, the emission ratio as a function of ADL concentration negates the influence of the substrate concentration and the reaction conditions.

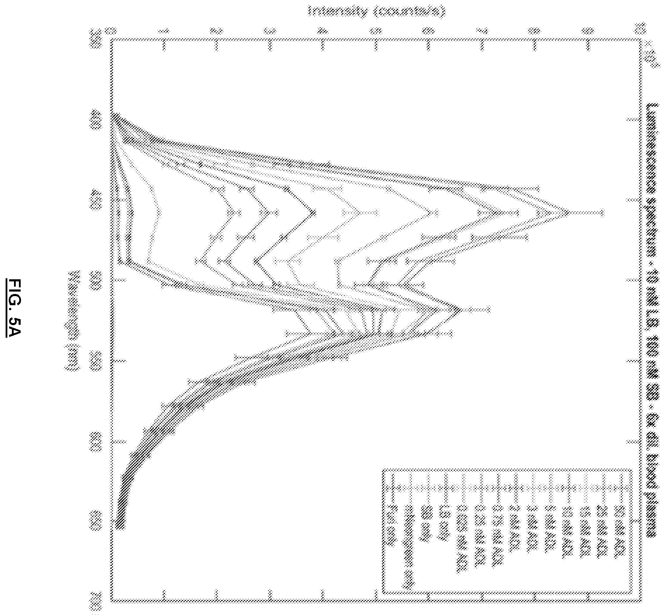

Figures 5A-B show an ADL titration experiment in 1:5 diluted blood plasma using 100 pM of NanoLuc-mNeonGreen as a calibrator luciferase in addition to 10 nM anti-AT-LB and 100 nM TNFα-SB. Figure 5A shows that the blue peak of reconstituted split Nanoluc increases as function of ADL concentration, whereas the peak at 513 nm from the calibrator luciferase remains constant. The titration curve obtained by plotting the ratio of the blue and green emission peaks as function of ADL concentration shows a large response in the physiologically relevant concentration range, showing a 15-20 fold overall change in emission ratio and a LOD of approximately 25 pM (corresponding to 0.15 nM in plasma).

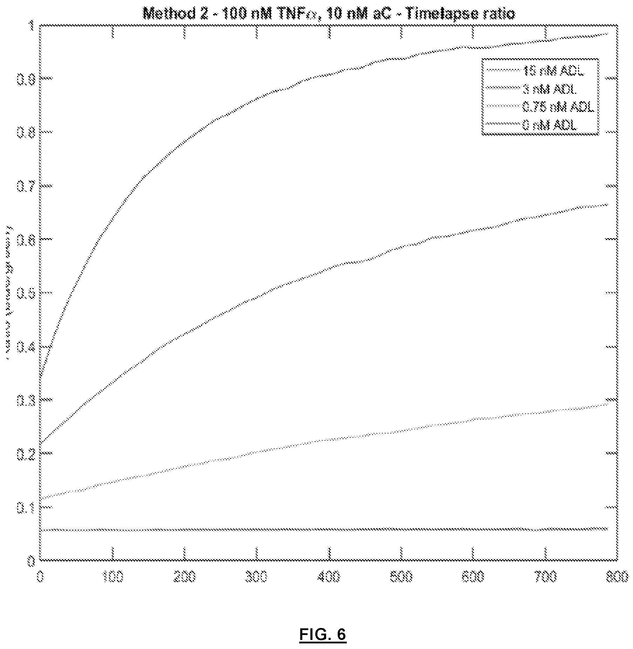

The ratiometric signal also allows one to reliably monitor the kinetics of complex formation, and thus establish the optimal incubation time. Figure 6 shows the increase in emission ratio following addition of 0, 0.75, 3 or 15 nM ADL to a mixture containing all the sensor components and substrate. As expected, the kinetics of complex formation are concentration dependent, reaching equilibrium at approx. 10 min for the higher concentrations. The emission ratio in the absence of ADL is constant.

### Conclusion

Given the above, a generic homogenous immunoassay format based on the complementation of split Nanoluc luciferase was developed for direct detection of TNFα-binding antibodies. Proof of concept was provided by successful ratiometric detection of adalimumab in diluted blood plasma with a LOD of 25 pM and a large dynamic range. In contrast to the sandwich immunoassay format based on two antibodies, the assay format introduced here has a fusion protein of the target antigen with a SB fragment of NanoLuc luciferase and a single detection antibody, which is conjugated to the LB fragment of Nanoluc, that binds to the adalimumab-TNFα complex. This new assay format is particularly attractive for the detection of therapeutic antibodies that bind to large and/or structurally complex discontinuous epitopes. The assay reported here could be easily amended for the detection of other TNFα-binding antibodies by using a detection antibody that is specific for the target antibody-TNFα-complex. The sensor principle could be further extended to other antibodies, provided an antigen-SB fusion protein can be produced and a specific detection antibody is available that either specifically binds the target antibody (but not at the antigen binding site), or binds to the complex of the target antibody and its antigen.

### Example 3. Development of red-shifted calibrator luciferase

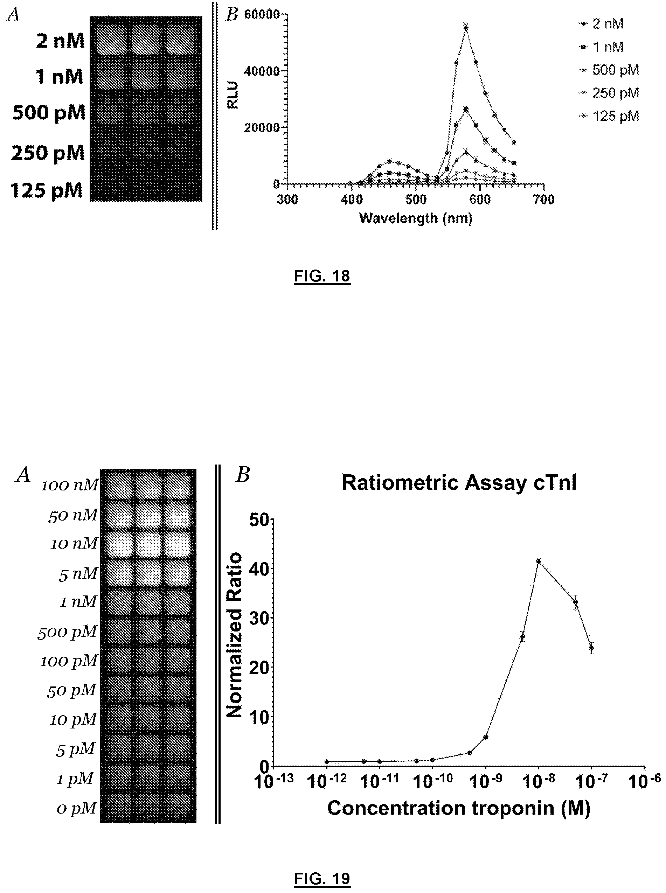

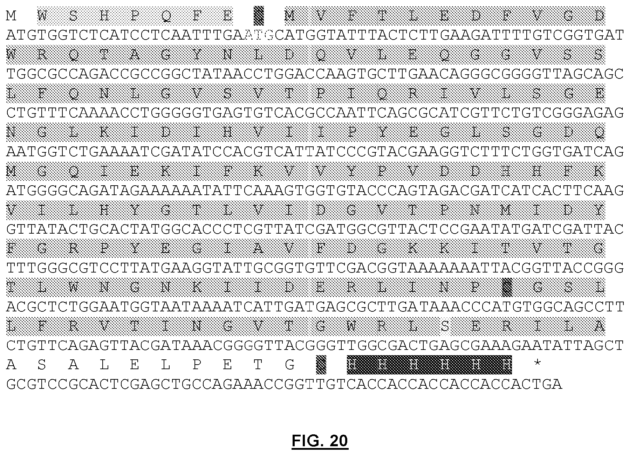

To develop a red-shifted NanoLuc luciferase, a fluorophore-labelled NanoLuc construct with highly efficient bioluminescence resonance energy transfer (BRET) from NanoLuc to the fluorophore was developed. The Cy3 fluorophore has an emission maximum at 563 nm and its excitation spectrum has spectral overlap with NanoLuc emission, and therefore was chosen to be incorporated into the NanoLuc luciferase via maleimide-thiol coupling. To achieve this, the native cysteine (Cys175) in NanoLuc was first replaced by serine, followed by site-directed mutagenesis to introduce a cysteine residue either near the N-terminus, C-terminus or in the accessible loops where the protein folding and bioluminescent properties are supposed not to be disrupted. The BRET efficiency was then determined for each mutant after labelling with Cy3, indicating K9C, D157C and G191C as potent Cy3-conjugation sites. In order to further improve the BRET efficiency, double and triple-site mutants by combing these mutation positions were subsequently constructed for coupling multiple Cy3 to one NanoLuc protein. The triple mutant K9C/D157C/G191C was eventually obtained with decent Cy3 labelling yield of approximately 140% and high BRET efficiency. The K9C/D157C/G191C variant emits orange luminescence (figure 18A) captured by a digital camera and has an emission maximum at 568 nm (figure 18B).

The Cy3-labeled K9C/D157C/G191C was then used as a calibrator luciferase to carry out a ratiometric assay of cTnl by using 19C7-LB and 4C2-SB sensor proteins. This red-shifted calibrator enabled a maximal ratio change of 45, superior to the green calibrator NL-mNG, which gave a maximal ratio change of 25 in the ratiometric assay of cTnl (figure 19).

### Description of the drawings

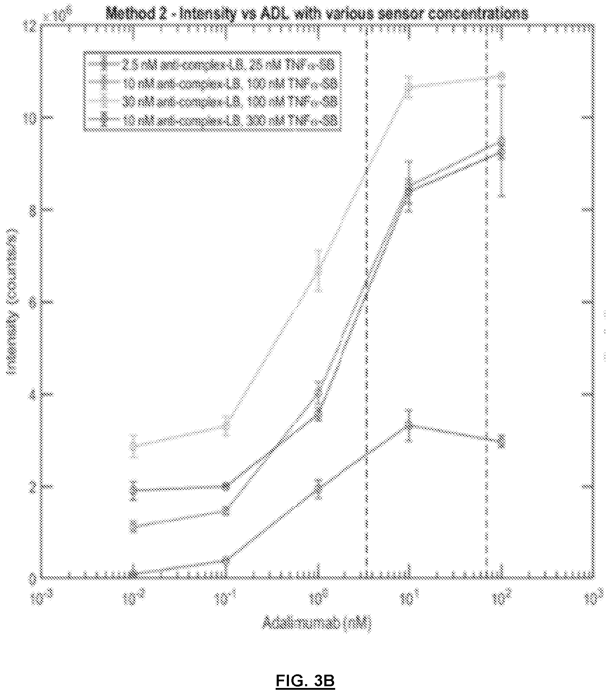

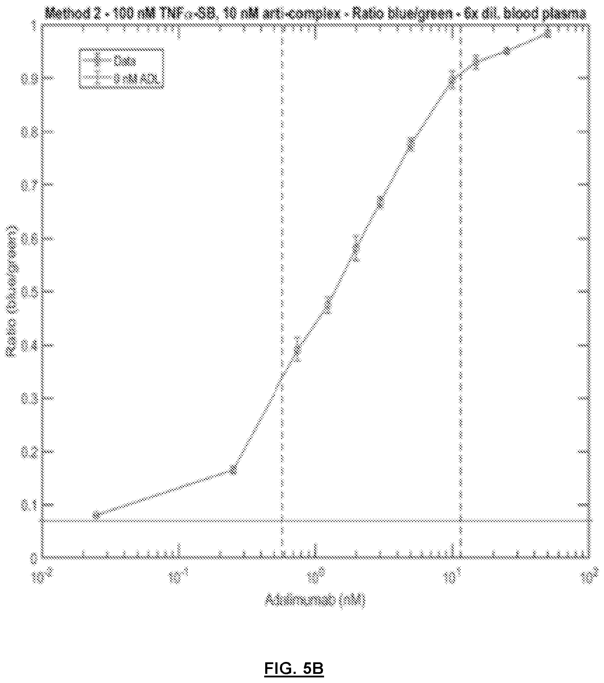

Figure 1A
   Schematic representation of homogeneous bioluminescent sandwich immunoassay. In the presence of antigen, antibody conjugated sensor proteins bind to the antigen, driving the reconstitution of active NanoLuc.
Figure 1B
   Split-luciferase based detection of TNFα binding antibodies. The SB fragment of Nanoluc is fused via a semi-flexible linker to recombinantly expressed TNFα, while the LB fragment is fused to photo-cross-linkable protein G, allowing conjugation to a detection antibody that specifically binds to the adalimumab-TNFα complex.
Figure 2
   SDS-PAGE analysis of TNFα-SB purification and antibody-pG-LB photo-crosslinking.
Figures 3A-B
   Intensiometric immunoassay for Adalimumab (figure 3A) Proof of principle using 2.5 nM anti-AT-LB and 25 nM TNFα-SB. Blue line indicates assay with all components, other colours indicate controls (figure 3B) adalimumab response curves using different concentrations of anti-AT-LB and TNFα-SB. All assays were done in PBS-BSA buffer using 500-fold diluted furimazine. Error bars represent mean ± SD (n=3).
Figure 4
   Intensiometric immunoassay for adalimumab in 1:3 diluted plasma using 10 nM anti-AT-LB and 100 nM TNFα-SB. Blue line indicates assay with all components, other colours indicate controls. All assays were done in PBS-BSA buffer mixed with blood plasma at a 1:3 ratio using 500-fold diluted furimazine. Error bars represent mean ± SD (n=3).
Figures 5A-B
   Ratiometric immunoassay for Adalimumab in 1:5 diluted plasma using 10 nM anti-AT-LB, 100 nM TNFα-SB and 100 pM NanoLuc-mNeonGreen. Emission spectra at different ADL concentrations (figure 5A). Emission ratio plotted as a function of ADL concentration (figure 5B). Blue line indicates assay with all components, other colors indicate controls. All assays were done in PBS-BSA buffer mixed with blood plasma at a 1:5 ratio using 500-fold diluted furimazine. Error bars represent mean ± SD (n=3).
Figure 6
   Ratiometric assay to monitor the kinetics of complex formation at various ADL concentrations. All assays were done using 10 nM anti-AT-LB, 100 nM TNFα-SB and 100 pM NanoLuc-mNeonGreen in PBS-BSA buffer mixed with blood plasma at a 1:5 ratio using 500-fold diluted furimazine.
Figure 7
   DNA and amino acid sequence of pG-LB. Strep-tag (gray), protein G (red), amber stop codon (yellow), LB (cyan), His-tag (dark red).
Figure 8
   DNA and amino acid sequence of His-SUMO-TNFα-SB. His-tag (dark red), SUMO-tag (yellow), TNFα (pink), SB (cyan), Strep-tag (gray).
Figure 9
   DNA and amino acid sequence of pG-SB. Strep-tag (gray), protein G (red), amber stop coden (yellow), SB (blue), His-tag (dark red).
Figure 10
   DNA and amino acid sequences of SB in pG-SB variants.
Figure 11
   Photo-conjugation of sensor proteins. Protein G-mediated site specific conjugation by UV illumination (figure 11A). Protein G contains the unnatural amino acid BPA (in red) and is fused to split NanoLuc (LB or SB) via a semiflexible linker. When bound to the Fc domain of antibody and activated by UV light, the protein G fusion protein is covalently conjugated to the antibody. Either one or two protein G adapters can be photo-crosslinked to the Fc domain. SDS-PAGE analysis of the purified photo-conjugated products (figure 11B). Lane 1, anti-cTnl 19C7 or 4C2; Lane 2, photo-conjugated products; Lane 3, purified photo-conjugated proteins.
Figure 12
   SDS-PAGE analysis of the photo-conjugated products by using different molar ratios of pGLB/SB to antibody. Lane 1, antibody; Lane 2, photo-conjugated products using equal molar ratio of antibody to pG-LB/SB; Lane 3, photo-conjugated products using 1:2 molar ratio of antibody to pG-LB/SB.
Figure 13
   Intensiometric immunoassay of cTnl. Figure 13A: cTnl dose-dependency using 1 nM 19C7-LB and 4C2-SB. Inset: picture of the samples. Figure 13B: limit of detection. Figure 13C: cTnl dose-dependency using different sensor concentrations. (■) 0.1 nM 19C7-LB and 0.1 nM 4C2-SB; (A) 0.1 nM 19C7-LB and 1 nM 4C2- SB; (•)1 nM 19C7-LB and 1 nM 4C2-SB; (◆) 1 nM 19C7-LB and 10 nM 4C2-SB. Figure 13D: time course of luminescence signal in the presence of different concentrations of cTnl. Error bars represent mean ± SD (n=3).
Figure 14
   Figure 14A: schematic representation of the ratiometric assays with NanoLuc-mNeonGreen fusion protein as a calibrator luciferase. Figure 14B: cTnl dose-dependency using 1 nM 19C7-LB and 4C2-SB spiked with 5 pM NanoLuc-mNeonGreen fusion protein. Inset: picture of the samples. Figure 14C: time course of emission ratio in the presence of different concentrations of cTnl.
Figure 15
   Bioluminescent immunoassay of CRP. Figure 15A: CRP dose-dependency using 1 nM C6-LB and 10 nM C135-SB. Figure 15B: time course of luminescence signal in the presence of different concentrations of CRP. Figure 15C: CRP dose-dependency using 1 nM C6-LB and 10 nM C135-SB spiked with 2 pM NanoLuc-mNeonGreen fusion protein in buffer. Figure 15D: time course of emission ratio in the presence of different concentrations of CRP.
Figure 16
   Correlation of CRP concentrations measured by ELISA and assay of the inventors. The samples were prepared in blood plasma, diluted 1/50 in buffer and quantified using calibration curves obtained in buffer. Error bars represent mean ± SD (n = 4).
Figure 17
   Bioluminescent immunoassays of anti-cetuximab. Figure 17A: intensiometric assays using 1 nM cetuximab-LB and cetuximab-SB. Figure 17B: kinetics of intensiometric assays with different concentrations of anti-cetuximab. Figure 17C: ratiometric assays using 1 nM cetuximab-LB and cetuximab-SB spiked with 1 pM NanoLuc-mNeonGreen fusion protein. Figure 17D: kinetics of ratiometric assays with different concentrations of anti-cetuximab.
Figure 18
   Luminescence of the red-shifted NanoLuc at different concentrations. Figure 18A: picture of taken by a digital camera; Figure 18B: luminescence spectra measured by a plate reader.
Figure 19
   Ratiometric assay of cardiac troponin I using 1 nM 19C7-LB and 4C2-SB spiked with 1 nM red-shifted NanoLuc. Figure 19A: picture of the samples taken by a digital camera; Figure 19B: normalized blue/red ratio measured by plate reader.
Figure 20
   DNA and amino acid sequence of K9C/D157C/G191C variant. Strep-tag (gray), NanoLuc (cyan), cysteine (red), His-tag (dark red).

## Claims

1. Bioluminescent assay for the ratiometric quantification of an analyte of interest, comprising a detector luciferase and a calibrator luciferase, wherein the detector luciferase:
- is responsive to the analyte of interest; and
- is reactive to a substrate to emit light at a first wavelength,
**characterised in that** the calibrator luciferase is functionalized with a fluorescent acceptor to allow energy transfer from the luciferase domain of the calibrator luciferase to the fluorescent acceptor, said calibrator luciferase:
- is non-responsive to the analyte of interest; and
- is reactive to the same substrate to emit light at a second wavelength which second wavelength is different from the first wavelength emitted by the detector luciferase, and
**in that** the detector luciferase and the calibrator luciferase have the same luciferase domain.

2. Bioluminescent assay according to claim 1, wherein the bioluminescent assay is selected from the group consisting of bioluminescent sandwich immunoassay, competitive immunoassays, sensor proteins based on allosteric modulation of luciferase activity or reversible control a luciferase inhibition, transcription regulation assays, assays screening for protein-protein interactions, and DNA or RNA detection assays.

3. Bioluminescent assay according to claim 1 or 2, wherein the luciferase domain of the detector luciferase and the calibrator luciferase is selected from the group consisting of luciferase domains of NanoLuc luciferase, Firefly luciferase, Renilla luciferase, Gaussia luciferase, TurboLuc luciferase and Aluc luciferase.

4. Bioluminescent assay according to any of the preceding claims, wherein the luciferase domain of the detector luciferase is a split luciferase domain.

5. Bioluminescent assay according to any of the preceding claims, wherein the fluorescent acceptor is selected from the group consisting of an mNeonGreen protein, a fluorescent protein, a Cy3, a fluorescent dye, a fluorescent quantum dot, a fluorescent nanoparticle, or a carbon dot.

6. Bioluminescent assay according to any of the preceding claims, wherein the analyte of interest is selected from the group consisting of an antigen, an antibody, and a ligand.

7. Use of the bioluminescent assay according to any of the preceding claims in a method for quantifying an analyte of interest in a sample.

8. Use of a calibrator luciferase in the bioluminescent assay according to any of claims 1-7 in a method for quantifying an analyte of interest in a sample, wherein the calibrator luciferase is functionalized with a fluorescent acceptor to allow energy transfer from the luciferase domain of the calibrator luciferase to the fluorescent acceptor.

9. Method for quantifying an analyte of interest in a sample, comprising the steps of:
i) providing a sample comprising the analyte of interest;
ii) providing a detector luciferase and a calibrator luciferase, wherein the detector luciferase:
- is responsive to the analyte of interest; and
- is reactive to a substrate to emit light at a first wavelength;
iii) measuring the bioluminescence intensity of the detector luciferase and the bioluminescence intensity of the calibrator luciferase for the sample provided in step i);
iv) calibrating the ratio of the measured bioluminescence intensity of the detector luciferase and the bioluminescence intensity of the calibrator luciferase; and
v) quantifying the analyte of interest based on the ratio calibrated in step iv),
**characterised in that** the calibrator luciferase is functionalized with a fluorescent acceptor to allow energy transfer from the luciferase domain of the calibrator luciferase to the fluorescent acceptor, said calibrator luciferase:
- is non-responsive to the analyte of interest; and
- is reactive to the same substrate to emit light at a second wavelength which second wavelength is different from the first wavelength emitted by the detector luciferase, and
**in that** the detector luciferase and the calibrator luciferase have the same luciferase domain.

10. Method according to claim 9, wherein the luciferase domain of the detector luciferase and the calibrator luciferase is selected from the group consisting of luciferase domains of NanoLuc luciferase, Firefly luciferase, Renilla luciferase, Gaussia luciferase, TurboLuc luciferase and Aluc luciferase.

11. Method according to claim 9 or 10, wherein the luciferase domain of the detector luciferase is a split luciferase domain.

12. Method according to any of claims 9-11, wherein the fluorescent acceptor is selected from the group consisting of an mNeonGreen protein, a fluorescent protein, a Cy3, a fluorescent dye, a fluorescent quantum dot, a fluorescent nanoparticle, or a carbon dot.

## Patentansprüche

1. Biolumineszenz-Assay zur ratiometrischen Quantifizierung eines Analyten von Interesse, umfassend eine Detektor-Luciferase und eine Kalibrator-Luciferase, wobei die Detektor-Luciferase:
- auf den Analyten von Interesse anspricht; und
- gegenüber einem Substrat reaktiv ist, so dass Licht mit einer ersten Wellenlänge emittiert wird,
**dadurch gekennzeichnet, dass** die Kalibrator-Luciferase mit einem Fluoreszenzakzeptor funktionalisiert ist, so dass eine Energieübertragung von der Luciferasedomäne der Kalibrator-Luciferase auf den Fluoreszenzakzeptor ermöglicht wird, wobei die Kalibrator-Luciferase:
- nicht auf den Analyten von Interesse anspricht; und
- gegenüber dem gleichen Substrat reaktiv ist, so dass Licht mit einer zweiten Wellenlänge emittiert wird, wobei die zweite Wellenlänge von der von der Detektor-Luciferase emittierten ersten Wellenlänge verschieden ist, und
dadurch, dass die Detektor-Luciferase und die Kalibrator-Luciferase die gleiche Luciferasedomäne aufweisen.

2. Biolumineszenz-Assay nach Anspruch 1, wobei der Biolumineszenz-Assay aus der Gruppe bestehend aus Biolumineszenz-Sandwich-Immunoassay, kompetitiven Immunoassays, Sensorproteinen basierend auf allosterischer Modulation von Luciferase-Aktivität oder reversibler Kontrolle einer Luciferase-Hemmung, Transkriptionsregulation-Assays, Screening-Assays für Protein-Protein-Wechselwirkungen und DNA- oder RNA-Nachweis-Assays ausgewählt ist.

3. Biolumineszenz-Assay nach Anspruch 1 oder 2, wobei die Luciferasedomäne der Detektor-Luciferase und der Kalibrator-Luciferase aus der Gruppe bestehend aus Luciferasedomänen von NanoLuc-Luciferase, Firefly-Luciferase, Renilla-Luciferase, Gaussia-Luciferase, TurboLuc-Luciferase und Aluc-Luciferase ausgewählt ist.

4. Biolumineszenz-Assay nach einem der vorhergehenden Ansprüche, wobei es sich bei der Luciferasedomäne der Detektor-Luciferase um eine gespaltene Luciferasedomäne handelt.

5. Biolumineszenz-Assay nach einem der vorhergehenden Ansprüche, wobei der Fluoreszenzakzeptor aus der Gruppe bestehend aus einem mNeonGreen-Protein, einem Fluoreszenzprotein, einem Cy3, einem Fluoreszenzfarbstoff, einem Fluoreszenz-Quantenpunkt, einem Fluoreszenz-Nanopartikel oder einem Kohlenstoff-Punkt ausgewählt ist.

6. Biolumineszenz-Assay nach einem der vorhergehenden Ansprüche, wobei der Analyt von Interesse aus der Gruppe bestehend aus einem Antigen, einem Antikörper und einem Liganden ausgewählt ist.

7. Verwendung des Biolumineszenz-Assays nach einem der vorhergehenden Ansprüche bei einem Verfahren zur Quantifizierung eines Analyten von Interesse in einer Probe.

8. Verwendung einer Kalibrator-Luciferase in dem Biolumineszenz-Assay nach einem der Ansprüche 1-7 bei einem Verfahren zum Quantifizieren eines Analyten von Interesse in einer Probe, wobei die Kalibrator-Luciferase mit einem Fluoreszenzakzeptor funktionalisiert ist, so dass eine Energieübertragung von der Luciferasedomäne der Kalibrator-Luciferase auf den Fluoreszenzakzeptor ermöglicht wird.

9. Verfahren zum Quantifizieren eines Analyten von Interesse in einer Probe, umfassend die Schritte:
i) Bereitstellen einer Probe, die den Analyten von Interesse umfasst;
ii) Bereitstellen einer Detektor-Luciferase und einer Kalibrator-Luciferase, wobei die Detektor-Luciferase:
- auf den Analyten von Interesse anspricht; und
- gegenüber einem Substrat reaktiv ist, so dass Licht mit einer ersten Wellenlänge emittiert wird;
iii) Messen der Biolumineszenzintensität der Detektor-Luciferase und der Biolumineszenzintensität der Kalibrator-Luciferase für die in Schritt i) bereitgestellte Probe;
iv) Kalibrieren des Verhältnisses der gemessenen Biolumineszenzintensität der Detektor-Luciferase und der Biolumineszenzintensität der Kalibrator-Luciferase; und
v) Quantifizieren des Analyten von Interesse, bezogen auf das in Schritt iv) kalibrierte Verhältnis,
**dadurch gekennzeichnet, dass** die Kalibrator-Luciferase mit einem Fluoreszenzakzeptor funktionalisiert ist, so dass eine Energieübertragung von der Luciferasedomäne der Kalibrator-Luciferase auf den Fluoreszenzakzeptor ermöglicht wird, wobei die Kalibrator-Luciferase:
- nicht auf den Analyten von Interesse anspricht; und
- gegenüber dem gleichen Substrat reaktiv ist, so dass Licht mit einer zweiten Wellenlänge emittiert wird, wobei die zweite Wellenlänge von der von der Detektor-Luciferase emittierten ersten Wellenlänge verschieden ist, und
dadurch, dass die Detektor-Luciferase und die Kalibrator-Luciferase die gleiche Luciferasedomäne aufweisen.

10. Verfahren nach Anspruch 9, wobei die Luciferasedomäne der Detektor-Luciferase und der Kalibrator-Luciferase aus der Gruppe bestehend aus Luciferasedomänen von NanoLuc-Luciferase, Firefly-Luciferase, Renilla-Luciferase, Gaussia-Luciferase, TurboLuc-Luciferase und Aluc-Luciferase ausgewählt ist.

11. Verfahren nach Anspruch 9 oder 10, wobei es sich bei der Luciferasedomäne der Detektor-Luciferase um eine gespaltene Luciferasedomäne handelt.

12. Verfahren nach einem der Ansprüche 9-11, wobei der Fluoreszenzakzeptor aus der Gruppe bestehend aus einem mNeonGreen-Protein, einem Fluoreszenzprotein, einem Cy3, einem Fluoreszenzfarbstoff, einem Fluoreszenz-Quantenpunkt, einem Fluoreszenz-Nanopartikel oder einem Kohlenstoff-Punkt ausgewählt ist.

## Revendications

1. Essai bioluminescent pour la quantification ratiométrique d'un analyte d'intérêt, comprenant une luciférase de détection et une luciférase d'étalonnage, dans lequel la luciférase de détection :
- est sensible à l'analyte d'intérêt ; et
- est réactive à un substrat pour émettre de la lumière à une première longueur d'onde,
**caractérisé en ce que** la luciférase d'étalonnage est fonctionnalisée avec un accepteur fluorescent pour permettre le transfert d'énergie du domaine luciférase de la luciférase d'étalonnage à l'accepteur fluorescent, ladite luciférase d'étalonnage :
- n'est pas sensible à l'analyte d'intérêt ; et
- est réactive au même substrat pour émettre de la lumière à une seconde longueur d'onde, laquelle seconde longueur d'onde est différente de la première longueur d'onde émise par la luciférase de détection, et
**en ce que** la luciférase de détection et la luciférase d'étalonnage ont le même domaine luciférase.

2. Essai bioluminescent selon la revendication 1, dans lequel l'essai bioluminescent est choisi dans le groupe constitué par un immunoessai en sandwich bioluminescent, des immunoessais compétitifs, des protéines de détection basées sur la modulation allostérique de l'activité luciférase ou le contrôle réversible d'une inhibition de la luciférase, des essais de régulation de la transcription, des essais de criblage des interactions protéine-protéine et des essais de détection d'ADN ou d'ARN.

3. Essai bioluminescent selon la revendication 1 ou 2, dans lequel le domaine luciférase de la luciférase de détection et de la luciférase d'étalonnage est choisi dans le groupe constitué par les domaines luciférase de la luciférase NanoLuc, de la luciférase de luciole, de la luciférase de Renilla, de la luciférase de Gaussia, de la luciférase TurboLuc et de la luciférase Aluc.

4. Essai bioluminescent selon l'une des revendications précédentes, dans lequel le domaine luciférase de la luciférase de détection est un domaine luciférase fractionné.

5. Essai bioluminescent selon l'une des revendications précédentes, dans lequel l'accepteur fluorescent est choisi dans le groupe constitué par une protéine mNeonGreen, une protéine fluorescente, Cy3, un colorant fluorescent, un point quantique fluorescent, une nanoparticule fluorescente ou un point de carbone.

6. Essai bioluminescent selon l'une des revendications précédentes, dans lequel l'analyte d'intérêt est choisi dans le groupe constitué par un antigène, un anticorps et un ligand.

7. Utilisation de l'essai bioluminescent selon l'une des revendications précédentes dans un procédé de quantification d'un analyte d'intérêt dans un échantillon.

8. Utilisation d'une luciférase d'étalonnage dans l'essai bioluminescent selon l'une des revendications 1 à 7 dans un procédé de quantification d'un analyte d'intérêt dans un échantillon, où la luciférase d'étalonnage est fonctionnalisée avec un accepteur fluorescent pour permettre le transfert d'énergie du domaine luciférase de la luciférase d'étalonnage à l'accepteur fluorescent.

9. Procédé de quantification d'un analyte d'intérêt dans un échantillon, comprenant les étapes :
i) de fourniture d'un échantillon comprenant l'analyte d'intérêt ;
ii) de fourniture d'une luciférase de détection et d'une luciférase d'étalonnage, où la luciférase de détection :
- est sensible à l'analyte d'intérêt ; et
- est réactive à un substrat pour émettre de la lumière à une première longueur d'onde ;
iii) de mesure de l'intensité de bioluminescence de la luciférase de détection et de l'intensité de bioluminescence de la luciférase d'étalonnage pour l'échantillon fourni dans l'étape i) ;
iv) d'étalonnage du rapport de l'intensité de bioluminescence mesurée de la luciférase de détection et de l'intensité de bioluminescence de la luciférase d'étalonnage ; et
v) de quantification de l'analyte d'intérêt sur la base du rapport étalonné dans l'étape iv),
**caractérisé en ce que** la luciférase d'étalonnage est fonctionnalisée avec un accepteur fluorescent pour permettre le transfert d'énergie du domaine luciférase de la luciférase d'étalonnage à l'accepteur fluorescent, ladite luciférase d'étalonnage :
- n'est pas sensible à l'analyte d'intérêt ; et
- est réactive au même substrat pour émettre de la lumière à une seconde longueur d'onde, laquelle seconde longueur d'onde est différente de la première longueur d'onde émise par la luciférase de détection, et
**en ce que** la luciférase de détection et la luciférase d'étalonnage ont le même domaine luciférase.

10. Procédé selon la revendication 9, dans lequel le domaine luciférase de la luciférase de détection et de la luciférase d'étalonnage est choisi dans le groupe constitué par les domaines luciférase de la luciférase NanoLuc, de la luciférase de luciole, de la luciférase de Renilla, de la luciférase de Gaussia, de la luciférase TurboLuc et de la luciférase Aluc.

11. Procédé selon la revendication 9 ou 10, dans lequel le domaine luciférase de la luciférase de détection est un domaine luciférase fractionné.

12. Procédé selon l'une des revendications 9 à 11, dans lequel l'accepteur fluorescent est choisi dans le groupe constitué par une protéine mNeonGreen, une protéine fluorescente, Cy3, un colorant fluorescent, un point quantique fluorescent, une nanoparticule fluorescente ou un point de carbone.
